Europäisches Patentamt

European Patent Office

Office européen des brevets

(11) Publication number: **0 296 986**
**A1**

(12) **EUROPEAN PATENT APPLICATION**

(21) Application number: **88401621.3**

(22) Date of filing: **24.06.88**

(51) Int. Cl.⁴: **A 61 B 17/16**
**B 23 C 5/10**

(30) Priority: **25.06.87 FR 8708956**

(43) Date of publication of application:
**28.12.88 Bulletin 88/52**

(84) Designated Contracting States:
**BE CH DE ES GB IT LI NL SE**

(71) Applicant: **S.A. BENOIST GIRARD & CIE**
**113, rue Jean Marie Naudin**
**F-92220 Bagneux (FR)**

(72) Inventor: **Kerboull, Marcel**
**25, Avenue du Docteur Arnold Netter**
**F-75012 Paris (FR)**

(74) Representative: **Rinuy, Santarelli**
**14, avenue de la Grande Armée**
**F-75017 Paris (FR)**

(54) **Intramedullary reamer.**

(57) The present invention concerns a surgical tool which is an intramedullary reamer.

This reamer comprises a hollow body (10), preferably cylindrical, of stamped and perforated metal with projecting reliefs (15) the front end (11) of which is open and the rear end (12) of which comprises means (17) for attaching it to a rod (13) which may be rotated by a motor.

A reamer of this kind advantageously replaces a rasp for removing the spongy part of the medullary canal of a damaged bone intended to receive the stem of a prosthesis.

FIG.1

EP 0 296 986 A1

## Description

### Intramedullary reamer

The present invention concerns a surgical tool, to be more precise an intramedullary reamer particularly suitable for fitting femoral prostheses.

When a surgeon has to implant a bone prosthesis and in particular a joint prosthesis such as a femoral prosthesis, he must first insert the stem of the prosthesis into the medullary canal of the bone in question.

To achieve this, after ablation of the defective bone end the surgeon must be able to remove the spongy part of the bone situated in the medullary canal.

Until now this has been done using a rasp driven into the damaged bone with a hammer and which is movable for the most part longitudinally.

To avoid this brutal way of cleaning out the interior of the bone intended to receive a prosthesis stem it would be desirable for the surgeon to be able to use a scraping tool that could turn to enable the interior of the medullary canal of the bone to be scraped out.

A objective of the present invention is a tool of this kind.

To be more precise, this tool consists in an intramedullary reamer characterised in that it has a stamped and perforated metal hollow body with projecting reliefs, an open front end and a rear end comprising means for attachment to a rod coaxial with the hollow body.

The rod, which may be coupled to a motor, is used to rotate the reamer, the projecting reliefs situated on the perforations at the periphery of the hollow body coming into contact with the spongy inside part of the bone.

The spongy bone debris can easily pass through the perforations to the interior of the reamer and be removed therefrom through its open front end.

The present invention will now be described with reference to the appended drawings, in which:

- figure 1 shows a view in longitudinal cross-section of a reamer in accordance with the invention fitted with a rod screwed onto one end; and

- figures 2 and 3 are respectively views in axial cross-section on the lines A-A and B-B in figure 1.

The intramedullary reamer shown in the figures has a hollow body 10 of stamped and perforated metal with projecting reliefs, an open front end 11 and a rear end 12 attached to a rod 13 by appropriate connection means.

The hollow body 10, which is preferably a hollow cylinder, is formed from a hollowed out bar or from stamped sheet metal, generally of stainless steel, mild steel or a hard metal such as titanium. This hollowed out bar or sheet metal comprises a number of perforations 14.

These perforations are aligned along the generatrices of the hollow body and each of the perforations 14 comprises at the periphery thereof a relied 15 projecting towards the outside of the hollow body.

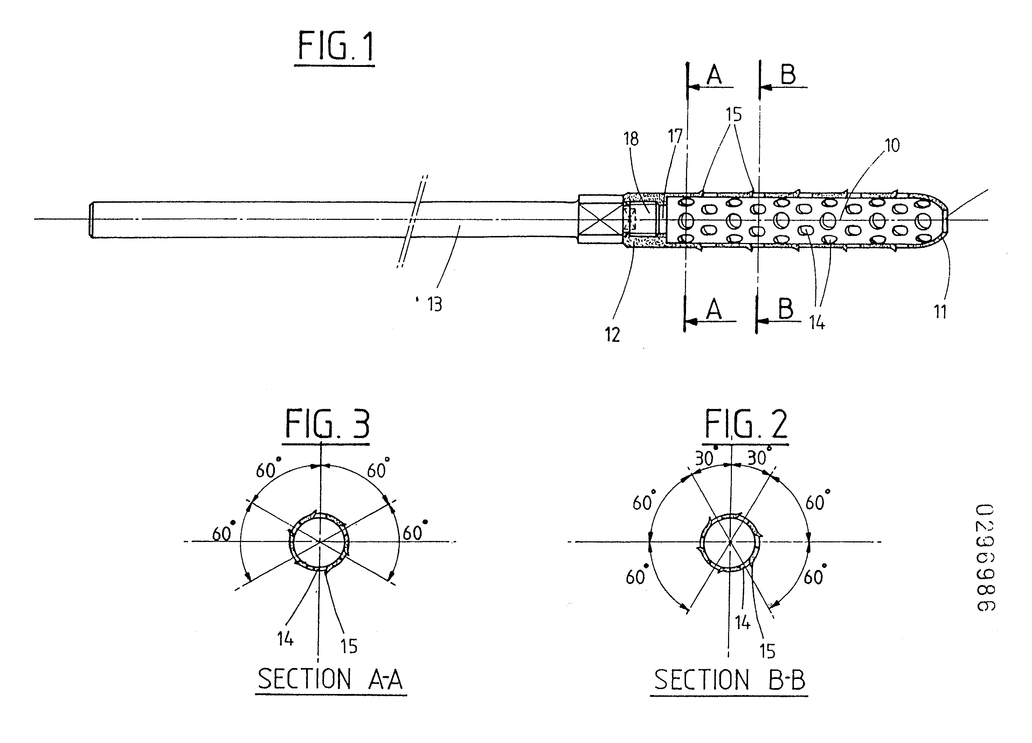

As shown in figure 1, the diameter of each of the perforations 14, which are of circular shape, passing through the projecting relief 15 is preferably at an angle to the axis of the reamer body and this angle is specifically 45 .

The size of the perforations 14 varies according to the diameter of the reamer. They generally have a diameter between 2 and 3 mm.

The perforations 14 are preferably offset from one generatrix to the next generatrix so as to be arranged quincunx fashion over the surface of the hollow body 10.

Thus a reamer with a diameter of 14 to 16 mm might have six peripheral perforations each with a diameter of 2.5 mm spaced at 60° from each other and offset by 30° from one axial cross-section to the next, so as to be arranged quincunx fashion, as shown in figures 2 and 3.

The perforations are usually formed along an even number of generatrices and this number, which depends on the size of the reamer, is generally equal to the diameter of the hollow tubular body in millimetres plus or minus 2 mm.

The perforations on the same generatrix are generally spaced by approximately 8 mm and the generatrices along which they are arranged quincunx fashion are generally separated by a circular arc length of approximately 3 mm.

The front end 11 has circular opening 16 coaxial with the hollow body but the diameter of which is less than that of the rear end of the hollow body, in particular within 10% of one-half that diameter.

The rear end 12 comprises means for attachment to the rod 13, which can be rotated by a motor.

The attachment means shown in figure 1 is a bore 17 internally screwthreaded which receives the externally screwthreaded end 18 of the rod 13.

Intramedullary reamers in accordance with the invention are preferably cylindrical with a length in the order of 60 mm, over 50 mm of which they are perforated, and their diameter may vary according to the size of the medullary canal of the bone to receive a prosthesis stem, specifically 10, 12, 14, 16 or 18 mm.

As shown in figure 1, the relief 15 projecting outwardly from each perforation 14 is at the upstream end thereof relative to the direction in which the rod 13 is screwed to the rear end 12 of the reamer.

The reamer in accordance with the invention may of course have shapes other than that shown in the appended figures.

Thus the hollow perforated metal body may be conical, although the cylindrical shape is particularly preferred.

Likewise, the connection between the rear end of the hollow body and the rod may be obtained by snap-action or the male and female screwthread members may be interchanged with the former on the rear end of the hollow body and the latter on the end of the rod.

## Claims

1. Intramedullary reamer comprising a perforated metal hollow body (10) with projecting reliefs, an open front end (11) and a rear end (12) with means for attachment to a rod (13) coaxial with the hollow body, characterised in that the hollow body (10) is made of stamped metal so that the perforations (14) each feature at the periphery thereof a relief (15) projecting towards the outside of the hollow body.

2. Reamer according to Claim 1 characterised in that the hollow body (10) is tubular.

3. Reamer according to Claim 1 or Claim 2 characterised in that the stamped metal hollow body (10) comprises a series of perforations (14) aligned along generatrices, said perforations being preferably distributed quincunx fashion over the outside surface of the body.

4. Reamer according to any one of Claims 1 to 3 characterised in that the perforations (14) in the hollow body (10) are of circular shape and the diameter of each perforation passing through the outwardly projecting relief (15) is at an angle to the axis of the body of the reamer and this angle is preferably 45°.

5. Reamer according to any one of Claims 1 through 4 characterised in that the perforations (14) in the hollow body (10) are aligned along an even number of generatrices of the hollow body, this number being preferably equal to the diameter of the hollow tubular body in millimetres plus or minus 2 mm.

6. Reamer according to any one of the preceding claims characterised in that the open front end (11) of the hollow body (10) comprises a circular opening (16) coaxial with the hollow body and of reduced diameter preferably equal to one-half the diameter of the rear end (12) of the hollow body plus or minus 10 %.

7. Reamer according to any one of the preceding claims characterised in that the means for attaching the rear end (12) to the rod (13) are operative by screwing.

8. Reamer according to claim 7 characterised in that the rear end (12) of the hollow body comprises a screwthreaded axial bore (17).

9. Reamer according to claim 7 characterised in that the outwardly projecting relief (15) of each perforation (14) is at the upstream end thereof relative to the direction in which the rod is screwed to the rear (12) of the reamer.

# FIG.1

# FIG.3

SECTION A-A

# FIG.2

SECTION B-B

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | FR-A-2 281 095 (WEIGAND) <br> * Figure 11; claim 1 * | 1,3,7,8 | A 61 B 17/16 <br> B 23 C 5/10 |
| Y | US-A-1 629 581 (MACHLET) <br> * Figures; page 1, line 107 – page 3, line 104 * | 1,3 | |
| A | | 5 | |
| Y | US-A-2 969 122 (STEFFES) <br> * Figures; column 2, lines 12-64 * | 7,8 | |
| A | DE-A-3 540 268 (STÜRMER) | | |
| A | FR-A-1 190 736 (SIMMONDS) | | |

TECHNICAL FIELDS SEARCHED (Int. Cl.4)

A 61 B
A 61 C
B 23 C
B 23 B
B 28 D
B 27 C
B 23 D

The present search report has been drawn up for all claims

| Place of search | Date of completion of the search | Examiner |
|---|---|---|
| THE HAGUE | 29-09-1988 | STEENBAKKER J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)